# EUROPEAN PATENT SPECIFICATION

(11) **EP 4 146 818 B1**
(45) Date of publication and mention of the grant of the patent: **10.09.2025**
(21) Application number: 21724258.5
(22) Date of filing: 06.05.2021
(51) Int. Cl.: C12Q 1/6816

(54) **REMOVAL OF EXCESS OLIGONUCLEOTIDES FROM A REACTION MIXTURE**
ENTFERNUNG VON ÜBERSCHÜSSIGEN OLIGONUKLEOTIDEN AUS EINEM REAKTIONSGEMISCH
ÉLIMINATION D'EXCÈS D'OLIGONUCLÉOTIDES À PARTIR D'UN MÉLANGE RÉACTIONNEL

(30) Priority: 08.05.2020 US 202063021875 P
(43) Date of publication of application: 15.03.2023
(73) Proprietor: F. Hoffmann-La Roche AG, 4070 Basel (CH); Roche Diagnostics GmbH, 68305 Mannheim (DE)
(72) Inventor: HIGGINS, Samuel, Pleasanton, California 94588 (US); O'HUALLACHAIN, Maeve, Pleasanton, California 94588 (US)
(74) Representative: Mockenhaupt, Stefan
(86) International application number: PCT/EP2021/061928
(87) International publication number: WO 2021/224359

(56) References cited:
- EP-A1- 2 204 458
- EP-A2- 2 308 997
- EP-B1- 2 308 997
- WO-A2-2006/044994
- CN-B- 104 342 486
- ES-A1- 2 421 459
- ES-B1- 2 421 459
- US-A1- 2019 194 748
- US-B2- 8 828 688

## Description

### FIELD OF THE INVENTION

The invention relates to the field of nucleic acids. More specifically, the invention relates to the field of *in vitro* reactions including nucleic acids.

### BACKGROUND OF THE INVENTION

Reactions utilizing short strands of nucleic acid ("oligonucleotides") often end with excess oligonucleotides left in the reaction mixture. These oligonucleotides can impede downstream processes by interacting with each other or off-target sample nucleic acids. Such interactions deplete essential reagents and impede desired reactions.

Excess oligonucleotides include primers, probes and adaptors. For example, amplification reactions (including polymerase chain reaction (PCR) and ligation chain reaction (LCR)) utilize oligonucleotides primers and probes. Excess primers and probes not incorporated into amplification products need to be removed prior to downstream processing.

Single molecule massively parallel sequencing involves forming nucleic acid libraries by attaching oligonucleotide adaptors with universal priming binding sites and other necessary features to the ends of each nucleic acid in the library. After the library is formed, excess adaptors not incorporated into the library nucleic acids need to be removed.

In the field of single cell analysis, U.S. Patent 10,144,950 describes a novel process of single-cell analysis called Quantum Barcoding (QBC), where each cell is labeled with a unique combinatorial barcode. The combinatorial barcode is assembled from subcode oligonucleotides. At the end of the code assembly process, the excess subcodes need to be removed.

CN 104342486 A relates to a miRNA detection method including hybridization on a miRNA-containing sample and a double hairpin nucleic acid hybridization probe to form a first compound; followed by restriction digestion and detecting the miRNA via PCR amplification.

US 2019/0194748 A1 relates to methods and probes for detection of nucleic acid target fragments. A number of possible designs of the probe are contemplated. For example, the 5' and 3' ends for ligation to the target fragment may be provided by head and tail sequences on two separate backbone oligonucleotides, or by head and tail sequences at respective ends of a single backbone oligonucleotide which loops to position the target fragment between the 5' and 3' ends.

WO 2006/044994 A2 relates to methods of nucleic acid amplification and detecting amplified products, and inter alia describes some methods of avoiding mis-priming with "leftover" primers.

ES 2 421 459 A1 describes removal of excess primers prior to amplification and/or sequencing through a hybridization capture step (i.e. a purification step). To effectuate capture of these unwanted excess primers and to prevent interference with any downstream detection steps, ES 2 421 459 A1 describes a purification process which utilizes forward and reverse primers each including a "fishing sequence".

EP 2308997 A2 is directed to a target-specific hybrid capture method, and describes that "blocker probes," which include oligonucleotides complementary to capture sequence probes, are used to eliminate capture sequence probe[s].

Typically, the removal involves a multi-step process including precipitation, centrifugation or bead capture. These steps consume time and reagents and cause the loss of target nucleic acids being separated from the excess oligonucleotides. Therefore, there is a need for a practical and economical method of removing excess oligonucleotides from reaction mixtures.

### SUMMARY OF THE INVENTION

The invention is set out in the appended claims, and relates to a method of removing excess oligonucleotides from a reaction mixture by capturing and sequestering them with a double-hairpin nucleic acid as defined by the claims. The excess oligonucleotides are ligated to the double hairpin structure forming a topologically circular closed nucleic acid strand that does not interfere with downstream applications.

In some embodiments, the invention is a method of removing undesired oligonucleotides from a reaction mixture, the method comprising: contacting the reaction mixture with a double hairpin nucleic acid comprising a single nucleic acid strand having: a first hairpin at the 5'-end; a second hairpin at the 3'-end; and a single-stranded region between the 5'-end and the 3'-end, wherein the single-stranded region comprises a sequence capable of hybridizing to the oligonucleotide to be removed; annealing the oligonucleotide to be removed to the double hairpin nucleic acid; ligating the oligonucleotide to be removed to the ends of the double hairpin nucleic acid to form an inert circular product, thereby removing the undesired oligonucleotide from the reaction mixture. The method may further comprise contacting the reaction mixture with a ligase. In other embodiments, the reaction mixture already comprises a ligase. In some embodiments, the double hairpin contains a 5'-phosphate group.

In some embodiments, the single stranded region of the double hairpin oligonucleotide comprises two regions of complementarity to the oligonucleotide to be removed flanking a single middle region. The middle region may be a non-nucleotide spacer or a region comprising inosine nucleotides. In some embodiments, the oligonucleotide to be removed comprises a plurality of oligonucleotides having two constant regions flanking a single barcode region varying among the plurality of oligonucleotides.

In some embodiments, the invention is a method of detecting a plurality of targets in a plurality of cells in a reaction mixture, the method comprising: binding to the targets in a plurality of cells a plurality of unique binding agents that are each specific for one of the targets; adding multiple subcode oligonucleotides to each of the bound agents in the plurality of cells in an ordered manner during successive rounds of split pool synthesis wherein the subcode oligonucleotides in each round anneal adjacently to the subcode oligonucleotide from a previous round via an annealing region, and covalently linking the adjacently annealed subcode oligonucleotides to each other to create in each cell, a unique cell-originating nucleotide code; contacting the reaction mixture with a double hairpin nucleic acid comprising a single nucleic acid strand having a first hairpin at the 5'-end; a second hairpin at the 3'-end; and a single-stranded region between the 5'-end and the 3'-end capable of hybridizing to the subcode oligonucleotides, and ligating excess subcode oligonucleotides to the corresponding double hairpin nucleic acid to form an inert circular product, thereby removing excess subcode oligonucleotides. The method may wherein further comprise contacting the sample with a polynucleotide kinase. In some embodiments, the subcode oligonucleotides comprise a barcode region flanked by two annealing regions and the single-stranded region of the double hairpin nucleic acid comprises a spacer equal in length to the barcode region flanked by two sequences capable of hybridizing to the annealing regions in the subcode oligonucleotides. The spacer may be a carbon linker or may comprise inosine-containing nucleotides.

In some embodiments, the invention is a method of preparing a solution of amplified target nucleic acids free of excess amplification primers, the method comprising: contacting a reaction mixture containing target nucleic acids with a forward and a reverse amplification primers and a thermostable nucleic acid polymerase in the presence of reagents supporting nucleic acid synthesis; subjecting the reaction mixture to a thermocycling profile suitable for annealing and extension of the forward and reverse primers; after the completion of the thermocycling profile, contacting the reaction mixture with a nucleic acid ligase and a double hairpin nucleic acids consisting of a single nucleic acid strand having: a first hairpin at the 5'-end; a second hairpin at the 3'-end; and a single-stranded region between the 5'-end and the 3'-end, wherein the single-stranded region is capable of hybridizing to the forward primer or to the reverse primer; ligating the forward and reserve primers to the corresponding double hairpin nucleic acid to form an inert circular product, thereby removing the forward and reverse primers from the solution of amplified target nucleic acids. The method may further comprise contacting the sample with a polynucleotide kinase.

In some embodiments, the invention is a method of preparing a solution of amplified target nucleic acids free of excess probes, the method comprising: contacting a reaction mixture containing target nucleic acids with a first and second probe capable of hybridizing adjacently to the target nucleic acid, and a thermostable ligase in the presence of reagents supporting ligation; subjecting the reaction mixture to a thermocycling profile suitable for annealing and ligation of the first and second probes to each other; after the completion of the thermocycling profile, contacting the reaction mixture with a double hairpin nucleic acids consisting of a single nucleic acid strand having: a first hairpin at the 5'-end; a second hairpin at the 3'-end; and a single-stranded region between the 5'-end and the 3'-end, wherein the single-stranded region is capable of hybridizing to the first probe or to the second probe; ligating the first and second probes to the corresponding double hairpin nucleic acid to form an inert circular product, thereby removing the first and second probes from the solution of amplified target nucleic acids. The method may further comprise contacting the sample with a polynucleotide kinase.

In some embodiments, the invention is a method of forming a library of nucleic acids free of excess adaptor molecules, the method comprising: contacting a reaction mixture comprising nucleic acids with adaptor molecules and a ligase; ligating the adaptor molecules to the ends of the nucleic acids; contacting the reaction mixture with a double hairpin nucleic acids consisting of a single nucleic acid strand having: a first hairpin at the 5'-end; a second hairpin at the 3'-end; and a single-stranded region between the 5'-end and the 3'-end, wherein the single-stranded region is capable of hybridizing to the adaptor; ligating the adaptor to the double hairpin nucleic acid to form an inert circular product, thereby removing the adaptor from the solution of amplified target nucleic acids. In some embodiments, the adaptor consists of two oligonucleotides forming at least one double-stranded region. In some embodiments, the reaction mixture is subjected to elevated temperature sufficient to separate the adaptor into single strands. In some embodiments, the double hairpin nucleic acid comprises a mixture of two double hairpin nucleic acids, each having the single stranded region complementary to one strand of the adaptor.

### BRIEF DESCRIPTION OF THE DRAWINGS

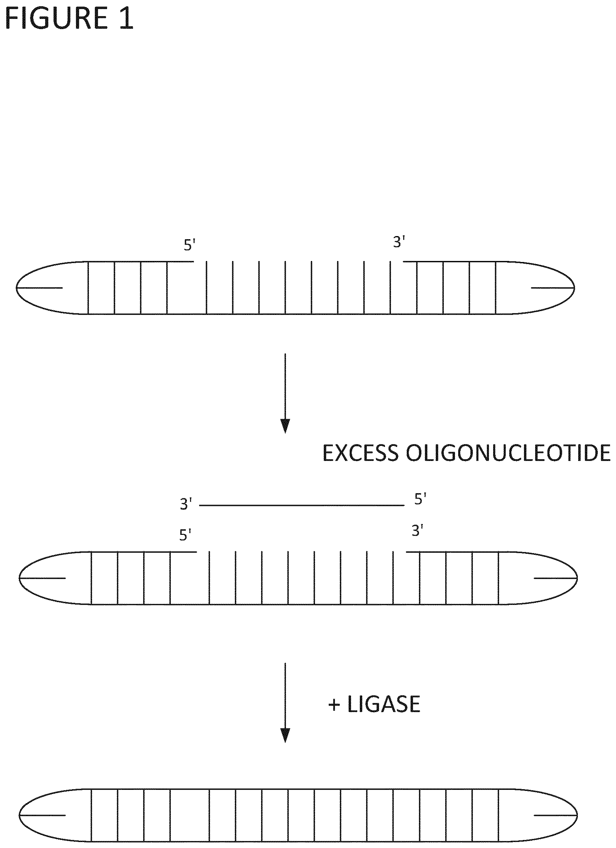

Figure 1 is a diagram of the double hairpin structure and method of use.

### DETAILED DESCRIPTION OF THE INVENTION

The term "nucleic acid" refers to a nucleotide polymer, and unless otherwise limited, includes known analogs of natural nucleotides that can function in a similar manner (e.g., hybridize) to naturally occurring nucleotides.

The terms "nucleotide sequence" and "nucleic acid" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, intergenic DNA, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), small nucleolar RNA, ribozymes, complementary DNA (cDNA), which is a DNA representation of mRNA, usually obtained by reverse transcription of messenger RNA (mRNA) or by amplification; DNA molecules produced synthetically or by amplification, genomic DNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non nucleotide components. A polynucleotide may be further modified after polymerization, such as by conjugation with a labeling component. Polynucleotide sequences, when provided, are listed in the 5' to 3' direction, unless stated otherwise. The term "oligonucleotide" refers to a shorter nucleic acid, typically no more than 100 nucleotides long although longer nucleic acids may also be called oligonucleotides.

A nucleic acid "probe" is an oligonucleotide capable of binding to a target nucleic acid of complementary sequence through one or more types of chemical bonds, generally through complementary base pairing, usually through hydrogen bond formation, thus forming a duplex structure. The probe binds or hybridizes to a "probe binding site." The probe can be labeled with a detectable label to permit facile detection of the probe, particularly once the probe has hybridized to its complementary target. Alternatively, however, the probe may be unlabeled, but may be detectable by specific binding with a ligand that is labeled, either directly or indirectly.

The term "epitope" and "target molecule" are used interchangeably herein to refer to the molecule of interest (protein or nucleic acid) being detected and/or quantified by the methods described herein.

The term "target oligonucleotide" refers to an oligonucleotide to be removed or depleted from a reaction mixture by the novel method disclosed herein. The target oligonucleotide may share similarities (e.g., share some nucleic acid sequence) with the target molecule defined above, however targeting the target oligonucleotide refers only to removing the target oligonucleotide from the reaction mixture according to the method disclosed herein. Target oligonucleotide is referred to interchangeably as "excess oligonucleotide," "undesired oligonucleotide," "oligonucleotide to be depleted" and "oligonucleotide to be removed."

The term "hairpin" in connection with nucleic acids refers to a secondary structure formed by a single strand of a nucleic acid having at least to portions complementary to each other and capable of annealing to each other. A hairpin comprises a short intervening region between the two complementary regions so that upon annealing, the nucleic acid strand makes a sharp bend. A hairpin with a longer intervening region does not form a sharp bend but instead forms a loop and the structure may be referred to as a "stem-loop" structure. There is no exact delineation between a hairpin and a stem-loop. A stem-loop structure with a smaller loop is sometimes referred to as a hairpin.

The invention is set out in the appended claims, and relates to an improved method or removing excess oligonucleotides or undesired oligonucleotides from reaction mixtures without any purification steps as defined by the claims. The method allows for easy removal or inactivation of oligonucleotides with little or no hands-on time and no loss of desired products.

In reactions involving mixtures of oligonucleotides, it is important to control and eliminate byproducts and excess reagents as they can serve as a substrate in problematic side reactions. Side reactions such as primer-primer or probe-probe interactions, off-target priming and off-target probe binding impact kinetics of intended reactions through sequestering reactants or consuming reaction components. The invention disclosed herein includes methods as defined by the claims rendering specific oligonucleotide sequences inert to side reactions and off-target base pairing.

The invention uses a short oligonucleotide (DNA or RNA) hereby referred to as a "double hairpin" (Figure 1). The double hairpin is added to the reaction mixture after completion of the reaction involving the oligonucleotide of which unused excess is to be removed. The sequence of this double hairpin oligonucleotide is designed such that it 1) forms hairpins at each of its ends and 2) has a middle section between the hairpins that binds to an oligonucleotide that is to be removed or rendered inert ("target oligonucleotide"). When the double hairpin oligonucleotide is added to the mixture containing the target oligonucleotide, the target oligonucleotide will anneal to the double hairpin's middle section. Upon annealing, a ligase enzyme is used to form a covalent bond between both the 5'- and the 3'-ends of the double hairpin oligonucleotide and both the 5'- and the 3'-ends of the target oligonucleotide, thus forming a topologically circular inert product.

The circularized product renders the target oligonucleotide inert in several ways. First, the ligation blocks the 3'-end of the target oligonucleotide from serving as a primer in any primer extension reaction, including exponential amplification reactions. Second, the base pairing with the double-hairpin oligonucleotide prevents the target oligonucleotide from base pairing with other nucleic acids in the reaction mixture.

The present invention involves a method as claimed of removing undesired oligonucleotides from a sample. In some embodiments, the sample is derived from a subject or a patient. In some embodiments the sample may comprise a fragment of a solid tissue or a solid tumor derived from the subject or the patient, *e.g.,* by biopsy. The sample may also comprise body fluids (*e.g.*, urine, sputum, serum, plasma or lymph, saliva, sputum, sweat, tear, cerebrospinal fluid, amniotic fluid, synovial fluid, pericardial fluid, peritoneal fluid, pleural fluid, cystic fluid, bile, gastric fluid, intestinal fluid, or fecal samples) that may contain nucleic acids. The sample may comprise whole blood or blood fractions where nucleic acids may be present. In other embodiments, the sample is a cultured sample, *e.g.,* a tissue culture containing cells from which nucleic acids may be isolated. In some embodiments, the nucleic acids of interest in the sample come from infectious agents such as viruses, bacteria, protozoa or fungi. In some embodiments, the sample cells are used in the procedure involving a target oligonucleotide to be removed. In other embodiments, isolated nucleic acids from the sample are used.

In some embodiments, nucleic acids need to be isolated from a sample. Methods of DNA extraction are well known in the art. See J. Sambrook et al., "Molecular Cloning: A Laboratory Manual," 1989, 2nd Ed., Cold Spring Harbor Laboratory Press: New York, N.Y.). A variety of kits are commercially available for extracting nucleic acids (DNA or RNA) from biological samples (e.g., BD Biosciences Clontech (Palo Alto, Cal.), Epicentre Technologies (Madison, Wisc.); Gentra Systems, INC. (Minneapolis, Minn.); and Qiagen, INC. (Valencia, Cal.), Ambion, Inc. (Austin, Tex.); BioRad Laboratories (Hercules, Cal.); and more.

In some embodiments, purification is by affinity binding. In variations of this embodiment, the affinity is to the specific target sequence (sequence capture). In other embodiments, the primer comprises an affinity tag. Any affinity tag known in the art can be used, such as biotin, an antibody, or an antigen for which a specific antibody exists. The affinity partner for the affinity tag may be present in solution, e.g., on a solution-phase solid support, such as suspended particles or beads, or bound to solid-phase support.

In some embodiments, nucleic acids are separated by size and purified using size exclusion chromatography, electophoresis or epitachophoresis.

Nucleic acids, proteins or other markers of interest may be present in the cells or reaction mixtures and may be the target of a detection or quantification procedure. The detection or quantification procedure is improved as disclosed herein by removal of excess oligonucleotides.

Each nucleic acid target is characterized by its nucleic acid sequence. Each protein target is characterized by its amino acid sequence and its epitopes recognized by specific antibodies. In some embodiments, the target nucleic acid contains a locus of a genetic variant, *e.g.,* a polymorphism, including a single nucleotide polymorphism or variant (SNP of SNV), or a genetic rearrangement resulting *e.g.,* in a gene fusion. In some embodiments, a protein biomarker contains an amino-acid change resulting in the creation of a unique epitope. In some embodiments, the target nucleic acid or target protein comprises a biomarker, *i.e.,* a gene or protein antigen whose variants are associated with a disease or condition. For example, the target nucleic acids and proteins can be selected from panels of disease-relevant markers described in U.S. Patent Application Ser. No. 14/774,518 filed on September 10, 2015. Such panels are available as AVENIO ctDNA Analysis kits (Roche Sequencing Solutions, Pleasanton, Cal.) In other embodiments, the target nucleic acids or proteins are characteristic of a particular organism and aids in identification of the organism or a characteristic of the pathogenic organism such as drug sensitivity or drug resistance. In yet other embodiments, the target nucleic acid or protein is a unique characteristic of a human subject, *e.g.,* a combination of HLA or KIR sequences defining the subject's unique HLA or KIR genotype. In yet other embodiments, the target nucleic acid is a somatic sequence such as a rearranged immune sequence representing an immunoglobulin (including IgG, IgM and IgA immunoglobulin) or a T-cell receptor sequence (TCR). In yet another application, the target is a fetal sequence present in maternal blood, including a fetal sequence characteristic of a fetal disease or condition or a maternal condition related to pregnancy. For example, the target could be one or more of the autosomal or X-linked disorders described in Zhang et al. (2019) Non-invasive prenatal sequencing for multiple Mendelian monogenic disorders using circulating cell-free fetal DNA, Nature Med. 25(3):439.

In some embodiments, the target is a nucleic acid (including mRNA, microRNA, viral RNA, cellular DNA or cell-free DNA (cfDNA) including circulating tumor DNA (ctDNA)).

In some embodiments, the target is a protein expressed in the cell. For example, the protein target may be cell-surface protein. In some embodiments, the cell surface protein is a lymphocyte surface protein selected from inhibitory receptors (such as Pdcd1, Havrcr2, Lag3, CD244, Entpd1, CD38, CD101, Tigit, CTLA4), cell surface receptors (such as TNFRSF9, TNFRSF4, Klrg1, CD28, Icos, IL2Rb, IL7R) or chemokine receptors (such as CX3CR1, CCL5, CCL4, CCL3, CSF1, CXCR5, CCR7, XCL1 and CXCL10). In some embodiments, the proteins are selected from CD4, CD8, CD11, CD16, CD19, CD20, CD45, CD56 and CD279.

Referring to Figure 1, the invention includes methods as claimed comprising the use of a double-hairpin oligonucleotide also referred to as a double hairpin nucleic acid for capturing undesired oligonucleotides from a reaction mixture. The oligonucleotide comprises three parts: a first hairpin at the 5'-end; a second hairpin at the 3'-end; and a single-stranded region between the 5'-end and the 3'-end hairpins, wherein the single-stranded region is capable of hybridizing to the oligonucleotide to be removed ("target oligonucleotide").

Each of the hairpins is formed by two sequences within the oligonucleotide that are capable of forming a stable hybrid with each other and forming a DNA (or RNA) bend between the hybridized sequences. In some embodiments, one or both hairpins are formed by regions of perfect complementarity with each other. In other embodiments, one or both hairpins are formed by only partially complementary sequences, which nevertheless, form a stable hairpin.

In some embodiments, one or both of the hairpin regions of the double hairpin oligonucleotide comprise one or more modified nucleotides increasing thermal stability (melting temperature, Tm) of the hairpin structures. For example, one or both hairpin portions include one or more of 5-methyl cytosine, 2,6-diaminopurine, 5-hydroxybutynl-2'-deoxyuridine, 8-aza-7-deazaguanosine, a ribonucleotide, a 2'O-methyl ribonucleotide or a locked nucleic acid.

In some embodiments, hairpin-forming sequences are artificial, optionally *in silico-designed* nucleic acid sequences. In other embodiments, hairpin-forming sequences are naturally occurring hairpin-forming sequences reviewed in Bikard et al., (2010) Folded DNA in Action: Hairpin Formation and Biological Functions in Prokaryotes, Microbiol. Mol. Biol. Review 74(4): 570-588; or in Brazda et al., (2011) Cruciform structures are a common DNA feature important for regulating biological process, BMC Mol. Biol. 12:33.

The single stranded portion of the hairpin is designed to form a stable hybrid with the target oligonucleotide to enable ligation of the target oligonucleotide with both ends of the double hairpin oligonucleotide. One of skill in the art would appreciate that a stable hybrid may be formed by nucleic acid strands that are perfectly complementary as well as less than perfectly complementary to each other. In some embodiments, the single-stranded region of the double hairpin oligonucleotide is partially complementary to the target oligonucleotide. In some embodiments, the single-stranded region of the double hairpin oligonucleotide is perfectly complementary to the target oligonucleotide. In some embodiments, a portion of the single-stranded region of the double hairpin oligonucleotide is perfectly complementary to the target oligonucleotide. In some embodiments, the 5'-end-adjacent portion and the 3'-end-adjacent portion of the single-stranded region are perfectly complementary to the 3'-end-adjacent portion and the 5'-end-adjacent portion of the target oligonucleotide, while the middle portion of the single-stranded region is not complementary or only partially complementary to the middle portion of the target oligonucleotide.

In some embodiments, the unfolded double hairpin oligonucleotide is between about 40 and about 200 nucleotides long. The hairpin-forming sequence is between about 9 and about 40 nucleotides long. While there is no prescribed length to either of the two hairpin regions, it is desired to have the regions long enough to ensure stability of the hairpins in the reaction mixture. One of skill in the art has access to a variety tools of predicting stability of a nucleic acid duplex (melting temperature, Tm) under various temperature and salt conditions. The single-stranded region is custom-made to accommodate the target oligonucleotide. Oligonucleotides including primers, probes and adaptors typically range from 10 to 100 nucleotides long with the majority falling between 20 and 50 nucleotides long. Therefore, the single stranded-region of the double hairpin oligonucleotide ranges from about 10 to about 100 nucleotides long, most often between 20 and 50 nucleotides long.

In some embodiments, the invention is a method as claimed of removing excess oligonucleotides or undesired oligonucleotides ("target oligonucleotides") from a reaction mixture. The method comprises contacting the reaction mixture containing the target oligonucleotides with a double hairpin nucleic acid comprising a single nucleic acid strand having: a first hairpin at the 5'-end; a second hairpin at the 3'-end; and a single-stranded region between the 5'-end and the 3'-end. The single-stranded region comprises a sequence capable of hybridizing to the target oligonucleotides.

The double hairpin oligonucleotide is added at a concentration calculated to exceed the concentration of the target oligonucleotide to be removed to ensure favorable kinetics of hybridization. One of skill in the art will appreciate that in some instances, the optimal molar concentration of double hairpin oligonucleotide can be calculated in advance, while in other instances, the optimal molar concentration of double hairpin oligonucleotide is determined experimentally through titration of the double hairpin oligonucleotide. One distinct advantage of the invention is that the double hairpin oligonucleotide is designed to interact with the target oligonucleotide and not interact with any other nucleic acid in the reaction mixture.

In some embodiments, the reaction mixture is incubated at a temperature optimal for the formation of the hybrid between the target oligonucleotide and the double hairpin oligonucleotide. In some embodiments, the annealing temperature is in the range of 40-72°C. In some embodiments, one or both of the hybridization step and the ligation step occur at ambient temperature.

In some embodiments, the reaction is allowed to incubate for the time needed for the formation of the hybrid between the target oligonucleotide and the double hairpin oligonucleotide. In some embodiments, the time is 5-15 minutes.

In some embodiments, a single target oligonucleotide is to be removed and a single double hairpin oligonucleotide is added. In other embodiments, multiple target oligonucleotides are to be removed and a mixture of multiple double hairpin oligonucleotides is added.

After the target oligonucleotide anneals to the single stranded region of the double hairpin oligonucleotide, the reaction mixture is contacted with a ligase. Ligase enzymes exists for ligating both DNA and RNA and will accommodate both DNA and RNA double hairpin oligonucleotide and target oligonucleotide. A ligase appropriate for the type of nucleic acid in the double hairpin oligonucleotide and the target oligonucleotide is used. Both ends of the target oligonucleotide are ligated to the double hairpin oligonucleotide (Figure 1).

In some embodiments, ligase and ligase cofactors and substrates (e.g., ATP) are already present in the reaction mixture due to the upstream process having taken place. In such embodiments, no additional ligase or ligase cofactors are added to remove the target oligonucleotide. In other embodiments, the ligase and ligase cofactors are added.

In some embodiments, both the target oligonucleotide and the double hairpin oligonucleotide contain 5'-phosphate groups. In other embodiments, one or both of the target oligonucleotide and the double hairpin oligonucleotide lack 5'-phosphate groups. In such embodiments, the reaction mixture is contacted with a polynucleotide kinase (PNK) and any necessary cofactors, e.g., ATP, and the 5'-ends are phosphorylated to enable ligation to take place.

In some embodiments, the enzymes (e.g., ligase and polynucleotide kinase) are removed or inactivated (e.g., heat inactivated) prior to downstream steps involving the reaction mixture depleted of target oligonucleotides.

One of skill in the art would appreciate that the term "removal" in reference to the target oligonucleotide includes all forms of inactivation of the target oligonucleotide. If the target oligonucleotide is no longer capable of hybridizing to any nucleic acids in the reaction mixture and is no longer capable of priming nucleic acid synthesis, the oligonucleotide is effectively removed from the reaction mixture. Therefore, sequestration of the target oligonucleotide within a double-stranded covalently closed topologically circular nucleic acid formed by the double hairpin oligonucleotide and the target oligonucleotide (Figure 1, bottom) constitutes removal of the target oligonucleotide from the reaction mixture.

In some embodiments, the method as claimed is used in an improved method of performing nucleic acid amplification via polymerase chain reaction (PCR). As described in detail in U.S. Patent No. 4,683,195, the polymerase chain reaction (PCR) involves contacting a sample solution comprising nucleic acids with a forward primer, a reverse primer, a nucleic acid polymerase, preferably a thermostable polymerase, and nucleic acid precursors such as dNTPs or dNTP analogs capable of being incorporated by the nucleic acid polymerase. The reaction mixture is subjected to a thermocycling profile including multiple cycles including a DNA denaturation step (90°C or higher), an optional primer annealing step (45-72°C) and a polymerase extension step (65-72°C). In some embodiments, PCR is real-time PCR including a fluorescently labeled detection probe as described e.g., in U.S. Patent 5,804,375. In state of the art protocols, after the amplification reaction has been completed, the reaction mixture is subjected to purification step, such as SPRI bead clean up, to remove excess primers. The instant invention as claimed dispenses with the purification step.

In some embodiments, after the completion of the amplification reaction, the reaction mixture is contacted with the novel double hairpin oligonucleotides described herein, according to the method as claimed. In some embodiments, the target oligonucleotide is a mixture of the forward and reverse primers. In some embodiments, the target oligonucleotide mixture also includes the detection probe. The double hairpin oligonucleotide is a combination of two or three oligonucleotides. The first double hairpin oligonucleotide includes a middle region complementary to the forward amplification primer. The second double hairpin oligonucleotide includes a middle region complementary to the reverse amplification primer. A third double hairpin oligonucleotide targeting the detection probe may also be present. The third double hairpin oligonucleotide includes a middle region complementary to the detection probe. The excess primers and if present, the probe, are ligated to the corresponding double-hairpin oligonucleotides to form closed circular structures.

In some embodiments, prior to ligation, the reaction mixture is contacted with a polynucleotide kinase (PNK) and any necessary cofactors and substrates, e.g., ATP, to phosphorylate the 5'-ends of the forward and reverse primers, and if present, the probe, to enable ligation to take place.

In some embodiments, double hairpin oligonucleotides are used for all three target oligonucleotides: the forward primer, the reverse primer and the probe. In other embodiments, double hairpin oligonucleotides are used for only one or two of the three target oligonucleotides.

Following the sequestration of the excess primers and if present, the excess probe in the closed circular structures, downstream steps can proceed immediately.

In some embodiments, the method as claimed is used in an improved method of performing nucleic acid amplification via ligase chain reaction (LCR). As described in detail in U.S. Patent No. 6,312,892, the ligase chain reaction (LCR) involves contacting a sample solution comprising nucleic acids with a probe set comprising the first and second oligonucleotide probe, the probes capable of hybridizing adjacently to the nucleic acid template, and a ligase, preferably a thermostable ligase. The reaction mixture is subjected to a thermocycling profile including multiple cycles including a DNA denaturation step (90°C or higher), an optional probe annealing step (45-65°C) and a ligation step (65°C or lower). In state of the art protocols, after the amplification reaction has been completed, the reaction mixture is subjected to a purification step, such as SPRI bead clean up, to remove excess probes. The instant invention as claimed dispenses with the purification step.

In some embodiments, after the completion of the amplification reaction, the reaction mixture is contacted with the novel double-hairpin oligonucleotides described herein, according to the method as claimed. In some embodiments, the target oligonucleotide is a mixture of the first and second probes. The double hairpin oligonucleotide is a combination of two oligonucleotides. The first double hairpin oligonucleotide includes a middle region complementary to the first probe. The second double hairpin oligonucleotide includes a middle region complementary to the second probe. Conveniently, the ligase and all the necessary cofactors are already present in the reaction mixture. The excess probes are ligated to the corresponding double hairpin oligonucleotides to form closed circular structures.

In some embodiments, prior to ligation, the reaction mixture is contacted with a polynucleotide kinase (PNK) and any necessary cofactors and substrates, e.g., ATP, to phosphorylate the 5'-ends of the probes to enable ligation to take place.

In some embodiments, double hairpin oligonucleotides are used for both probes. In other embodiments, double hairpin oligonucleotides are used for only one of the two probes.

Following the sequestration of the excess primers and if present, the probe in the closed circular structures, downstream steps can proceed immediately.

In some embodiments, the method as claimed is used in an improved method of performing quantum barcoding (QBC), a method of detecting multiple targets in a plurality of individual cells (U.S. Patent 10,144,950). The method comprises comprising preparing a cell suspension. Next, the cells are contacted with a unique binding agent, e.g., DNA probe or RNA probe (including an aptamer) or an antibody. The probe or antibody comprises at least one part or element specifically interacting with a target and an element allowing for assembly of a combinatorial nucleic acid barcode. In some embodiments, the assay is a multiplex assay, whereby a plurality of target molecules is detected in a plurality of cells in a single reaction mixture using a plurality of different binding agents of same or different kind (e.g., a plurality of different nucleic acid probes, or a plurality of different antibodies, or a combination of nucleic acid probes and antibodies). If the unique binding agent in an antibody, the antibody may comprise a linker oligonucleotide that facilitates assembly of a barcode. Methods to attach nucleic acids to antibodies are known, e.g., Gullberg et al., PNAS 101 (22): pages 228420-8424 (2004); Boozer et al, Analytical Chemistry, 76(23): pages 6967-6972 (2004) or Kozlov et al., Biopolymers 5: 73 (5): pages 621-630 (2004). If the unique binding agent is a nucleic acid probe, a barcode may be attached directly to the probe.

After the unique binding agent has bound to its target, the cells with bound probes or antibodies are subjected to a split-pool barcode assembly described in more detail in the U.S. Patent 10,144,950. A unique cell-originating code is assembled on each cell where a unique binding agent has bound. The unique cellular barcode is a modular structure assembled from subunits by stepwise addition of subunits. Each subunit comprises a barcode and attachment regions serving to attach the subunit to the growing unique cellular barcode. The subunits attach to each other or to a common backbone via attachment regions, e.g., complementary nucleic acid sequences. The attachment may comprise one or both of hybridization to the backbone or to the adjacent subunit and ligation to the adjacent subunit. After the unique cellular barcodes have been assembled, they are isolated from cells and detected. For example, the unique cellular barcodes are amplified and sequenced.

In one embodiment of the instant invention, prior to the amplification step, the reaction mixture containing the unique barcodes is contacted with the novel double-hairpin oligonucleotides described herein, according to the method as claimed. The target oligonucleotide is a mixture of the plurality of barcode subunits. The double hairpin oligonucleotide is designed to bind one or more including all the barcode subunits in the mixture. In some embodiments, the single-stranded portion of the double hairpin oligonucleotide comprises two regions capable of hybridizing to the annealing regions of the barcode subunits, flanking a single middle region having the length of the subcode contained in the barcode subunits. Because the subcodes vary among the barcode subunits, the middle region of the double hairpin oligonucleotide is designed to accommodate this diversity. In some embodiments, the middle region of the double hairpin oligonucleotide is a non-nucleotide spacer of the length corresponding to the length of the subcode or an inosine containing nucleic acid with the number of inosine nucleotides corresponding to the number of nucleotides in the subcode.

In some embodiments, the barcode subunit oligonucleotides are capable of being ligated into unique barcodes, i.e., already conveniently comprise a 5'-phosphate. Furthermore, in such embodiments an active ligase is already conveniently present in the reaction mixture. In such embodiments, the reaction mixture is contacted only with the double hairpin oligonucleotides.

In other embodiments, the barcode subunits are added to the unique barcode by a method other than ligation (see e.g., an embodiment where the barcodes are copied by a polymerase, *see* U.S. application Serial No. 16/250,974, filed on January 19, 2019). In such embodiments, the reaction mixture is contacted with the double hairpin oligonucleotides and is further contacted with a ligase. If the barcode subunits lack the 5'-phspohate, the reaction mixture is further contacted a polynucleotide kinase (PNK). In some embodiments, the reaction mixture is contacted with ATP the necessary cofactors for the kinase and the ligase.

The excess barcode subunits are ligated to the double-hairpin oligonucleotides to form a closed circular structure. Following the sequestration of the excess barcode subunit oligonucleotides in the closed circular structure, amplification can proceed immediately. The barcode oligonucleotides are no longer able to interfere with nucleic acid amplification, e.g., by serving as amplification primers.

It is especially advantageous that with the instant invention, no purification steps are needed prior to amplification. Quantum Bar Coding (QBC) is a method of single cell analysis where each unique cellular barcode represents a single cell. Therefore, a loss of a single unique cellular barcode molecule represents a loss of valuable data point. With the method described herein, after the removal of excess barcode subunits, the downstream analysis steps may proceed immediately.

In some embodiments, the method as claimed is used in an improved method of forming a library for nucleic acid sequencing. In this embodiment, nucleic acids in a sample are ligated to adaptors and excess adaptors are removed prior to downstream processing of the libraries.

In some embodiments, the method includes treating nucleic acids in the sample prior to ligating adaptors. The sample nucleic acids may already be blunt-ended or may be rendered blunt-ended by enzymatic treatment (e.g., "end repair"). In other embodiments, the blunt-ended DNA undergoes A-tailing where a single A nucleotide is added to the 3'-end of one or both blunt ends. The adaptors may be double-stranded or partially double-stranded short nucleic acids having one blunt end with a single T nucleotide extending from the blunt end to facilitate ligation between the sample nucleic acid and the adaptor. Commercially available kits for performing end repair, A-tailing and adaptor ligation include AVENIO ctDNA Library Prep Kit or KAPA HyperPrep and HyperPlus kits (Roche Sequencing Solutions, Pleasanton, Cal.).

In some embodiments, the instant invention includes a novel step of removing excess adaptors from a library forming reaction by sequestering them within a closed circular structure formed by ligation to a double-hairpin oligonucleotide described herein, according to the method as claimed. The target oligonucleotide includes both strands of the adaptor. The double hairpin is a combination of two double hairpin oligonucleotides. The first double hairpin oligonucleotide comprises a middle region complementary to the first strand of the adaptor while the second double hairpin oligonucleotide comprises a middle region complementary to the second strand of the adaptor.

In some embodiments, the method further includes a step of incubating the reaction mixture comprising the library and the excess adaptors at an elevated temperature sufficient to separate the strands of the adaptors but not the strands of the library nucleic acids or the hairpin structures in the double hairpin oligonucleotide. To facilitate this step of the method, the double hairpin oligonucleotide may comprise modified nucleotides increasing the melting temperature (Tm) of the hairpin. For example, one or both hairpin portions include one or more of 5-methyl cytosine, 2,6-diaminopurine, 5-hydroxybutynl-2'-deoxyuridine, 8-aza-7-deazaguanosine, a ribonucleotide, a 2'O-methyl ribonucleotide or a locked nucleic acid.

In some embodiments, the reaction mixture is also contacted with a polynucleotide kinase to phosphorylate the 5'-ends of the adaptors.

The method of the instant invention next comprises a step of ligating the adaptor strands to the double hairpin oligonucleotides. The method may further comprise amplifying the library or subjecting the library to a step of target capture. No purification steps are necessary to prior to the downstream steps of amplification or target capture.

The excess oligonucleotide removal method of the invention is not limited to the exemplary applications specifically addressed above but can be used in any diagnostic, prognostic, therapeutic, patient stratification, drug development, treatment selection, and screening process that involves the use of oligonucleotides where removing excess oligonucleotides is desired.

## Claims

1. A method of removing undesired oligonucleotides from a reaction mixture, the method comprising:
a. contacting the reaction mixture with a double hairpin nucleic acid comprising a single nucleic acid strand having:
i. a first hairpin at the 5'-end;
ii. a second hairpin at the 3'-end; and
iii. a single-stranded region between the 5'-end and the 3'-end, wherein the single-stranded region comprises a sequence capable of hybridizing to the oligonucleotide to be removed;
b. annealing the oligonucleotide to be removed to the double hairpin nucleic acid;
c. ligating the oligonucleotide to be removed to the ends of the double hairpin nucleic acid to form an inert circular product, thereby removing the undesired oligonucleotide from the reaction mixture.

2. The method of claim 1, further comprising contacting the reaction mixture with a ligase prior to step c.

3. The method of claim 1, wherein the reaction mixture comprises a ligase prior to step a.

4. The method of claim 1, wherein the double hairpin contains a 5'-phosphate group.

5. The method of claim 1, wherein the single stranded region comprises two regions of complementarity to the oligonucleotide to be removed flanking a single middle region.

6. The method of claim 5, wherein the middle region is a non-nucleotide spacer.

7. The method of claim 5, wherein the middle region comprises inosine nucleotides.

8. The method of claim 5, wherein the oligonucleotide to be removed comprises a plurality of oligonucleotides having two constant regions flanking a single barcode region varying among the plurality of oligonucleotides.

9. A method of detecting a plurality of targets in a plurality of cells in a reaction mixture, the method comprising:
a. binding to the targets in a plurality of cells a plurality of unique binding agents that are each specific for one of the targets;
b. adding multiple subcode oligonucleotides to each of the bound agents in the plurality of cells in an ordered manner during successive rounds of split pool synthesis wherein the subcode oligonucleotides in each round anneal adjacently to the subcode oligonucleotide from a previous round via an annealing region, and covalently linking the adjacently annealed subcode oligonucleotides to each other to create in each cell, a unique cell-originating nucleotide code;
c. contacting the reaction mixture with a double hairpin nucleic acid comprising a single nucleic acid strand having a first hairpin at the 5'-end; a second hairpin at the 3'-end; and a single-stranded region between the 5'-end and the 3'-end capable of hybridizing to the subcode oligonucleotides, and ligating excess subcode oligonucleotides to the corresponding double hairpin nucleic acid to form an inert circular product, thereby removing excess subcode oligonucleotides.

10. The method of claim 9, wherein the subcode oligonucleotides comprise a barcode region flanked by two annealing regions and the single-stranded region of the double hairpin nucleic acid comprises a spacer equal in length to the barcode region flanked by two sequences capable of hybridizing to the annealing regions in the subcode oligonucleotides.

11. A method of preparing a solution of amplified target nucleic acids free of excess amplification primers, the method comprising:
a. contacting a reaction mixture containing target nucleic acids with a forward and a reverse amplification primers and a thermostable nucleic acid polymerase in the presence of reagents supporting nucleic acid synthesis;
b. subjecting the reaction mixture to a thermocycling profile suitable for annealing and extension of the forward and reverse primers;
c. after the completion of the thermocycling profile, contacting the reaction mixture with a nucleic acid ligase and a double hairpin nucleic acids consisting of a single nucleic acid strand having:
i. a first hairpin at the 5'-end;
ii. a second hairpin at the 3'-end; and
iii. a single-stranded region between the 5'-end and the 3'-end, wherein the single-stranded region is capable of hybridizing to the forward primer or to the reverse primer;
d. ligating the forward and reserve primers to the corresponding double hairpin nucleic acid to form an inert circular product, thereby removing the forward and reverse primers from the solution of amplified target nucleic acids.

12. A method of preparing a solution of amplified target nucleic acids free of excess probes, the method comprising:
a. contacting a reaction mixture containing target nucleic acids with a first and second probe capable of hybridizing adjacently to the target nucleic acid, and a thermostable ligase in the presence of reagents supporting ligation;
b. subjecting the reaction mixture to a thermocycling profile suitable for annealing and ligation of the first and second probes to each other;
c. after the completion of the thermocycling profile, contacting the reaction mixture with a double hairpin nucleic acids consisting of a single nucleic acid strand having:
i. a first hairpin at the 5'-end;
ii. a second hairpin at the 3'-end; and
iii. a single-stranded region between the 5'-end and the 3'-end, wherein the single-stranded region is capable of hybridizing to the first probe or to the second probe;
d. ligating the first and second probes to the corresponding double hairpin nucleic acid to form an inert circular product, thereby removing the first and second probes from the solution of amplified target nucleic acids.

13. A method of forming a library of nucleic acids free of excess adaptor molecules, the method comprising:
a. contacting a reaction mixture comprising nucleic acids with adaptor molecules and a ligase;
b. ligating the adaptor molecules to the ends of the nucleic acids to form a library of nucleic acids;
c. contacting the reaction mixture with a double hairpin nucleic acids consisting of a single nucleic acid strand having:
i. a first hairpin at the 5'-end;
ii. a second hairpin at the 3'-end; and
iii. a single-stranded region between the 5'-end and the 3'-end, wherein the single-stranded region is capable of hybridizing to the adaptor;
d. ligating the adaptor to the double hairpin nucleic acid to form an inert circular product, thereby removing the adaptor from the solution containing the library of nucleic acids.

## Patentansprüche

1. Verfahren zum Entfernen unerwünschter Oligonukleotide aus einem Reaktionsgemisch, wobei das Verfahren Folgendes umfasst:
a. Inkontaktbringen des Reaktionsgemisches mit einer Doppelhaarnadelnukleinsäure, die einen Nukleinsäureeinzelstrang umfasst, der Folgendes aufweist:
i. eine erste Haarnadel am 5'-Ende;
ii. eine zweite Haarnadel am 3'-Ende und
iii.eine einzelsträngige Region zwischen dem 5'-Ende und dem 3'-Ende, wobei die einzelsträngige Region eine Sequenz umfasst, die an das zu entfernende Oligonukleotid hybridisieren kann;
b. Annealen des zu entfernenden Oligonukleotids an die Doppelhaarnadelnukleinsäure;
c. Ligieren des zu entfernenden Oligonukleotids an die Enden der Doppelhaarnadelnukleinsäure unter Bildung eines inerten zirkulären Produktes, wodurch das unerwünschte Oligonukleotid aus dem Reaktionsgemisch entfernt wird.

2. Verfahren nach Anspruch 1, ferner umfassend das Inkontaktbringen des Reaktionsgemisches mit einer Ligase vor Schritt c.

3. Verfahren nach Anspruch 1, wobei das Reaktionsgemisch vor Schritt a. eine Ligase umfasst.

4. Verfahren nach Anspruch 1, wobei die Doppelhaarnadel eine 5'-Phosphatgruppe enthält.

5. Verfahren nach Anspruch 1, wobei die einzelsträngige Region zwei Regionen von Komplementarität zu dem zu entfernenden Oligonukleotid umfasst, die eine einzelne mittlere Region flankieren.

6. Verfahren nach Anspruch 5, wobei die mittlere Region ein Nicht-Nukleotid-Spacer ist.

7. Verfahren nach Anspruch 5, wobei die mittlere Region Inosinnukleotide umfasst.

8. Verfahren nach Anspruch 5, wobei das zu entfernende Oligonukleotid eine Vielzahl von Oligonukleotiden umfasst, die zwei konstante Regionen aufweisen, die eine einzelne Barcode-Region flankieren, die unter der Vielzahl von Oligonukleotiden variiert.

9. Verfahren zum Nachweisen einer Vielzahl von Zielen in einer Vielzahl von Zellen in einem Reaktionsgemisch, wobei das Verfahren Folgendes umfasst:
a. Binden einer Vielzahl von spezifisch bindenden Mitteln, die jeweils für eines der Ziele spezifisch sind, an die Ziele in einer Vielzahl von Zellen;
b. Zugeben mehrerer Subcode-Oligonukleotide zu jedem der gebundenen Mittel in der Vielzahl von Zellen in geordneter Weise während aufeinanderfolgender Runden von Split-Pool-Synthese, wobei die Subcode-Oligonukleotide in jeder Runde über eine Annealing-Region angrenzend an das Subcode-Oligonukleotid aus einer vorhergehenden Runde annealen, und kovalentes Verknüpfen der angrenzend annealten Subcode-Oligonukleotide miteinander, um in jeder Zelle einen spezifischen Nukleotid-Code mit Zellursprung zu schaffen;
c. Inkontaktbringen des Reaktionsgemisches mit einer Doppelhaarnadelnukleinsäure, die einen Nukleinsäureeinzelstrang umfasst, der eine erste Haarnadel am 5'-Ende; eine zweite Haarnadel am 3'-Ende und eine einzelsträngige Region zwischen dem 5'-Ende und dem 3'-Ende, die an die Subcode-Oligonukleotide hybridisieren kann, aufweist und Ligieren überschüssiger Subcode-Oligonukleotide an die entsprechende Doppelhaarnadelnukleinsäure unter Bildung eines inerten zirkulären Produktes, wodurch überschüssige Subcode-Oligonukleotide entfernt werden.

10. Verfahren nach Anspruch 9, wobei die Subcode-Oligonukleotide eine Barcode-Region umfassen, die von zwei Annealing-Regionen flankiert wird, und die einzelsträngige Region der Doppelhaarnadelnukleinsäure einen Spacer umfasst, der die gleiche Länge hat, wie die Barcode-Region, und von zwei Sequenzen flankiert wird, die an die Annealing-Regionen in den Subcode-Oligonukleotiden hybridisieren können.

11. Verfahren zum Herstellen einer Lösung von amplifizierten Zielnukleinsäuren, die frei von überschüssigen Amplifikationsprimern ist, wobei das Verfahren Folgendes umfasst:
a. Inkontaktbringen eines Zielnukleinsäuren enthaltenden Reaktionsgemisches mit einem Vorwärts- und einem Rückwärtsamplifikationsprimer und einer wärmebeständigen Nukleinsäurepolymerase in der Gegenwart von Reagenzien, die die Nukleinsäuresynthese unterstützen;
b. Unterziehen des Reaktionsgemisches einem Thermocycling-Profil, das zum Annealen und Verlängern des Vorwärts- und Rückwärtsprimers geeignet ist;
c. nach Abschluss des Thermocycling-Profils, Inkontaktbringen des Reaktionsgemisches mit einer Nukleinsäureligase und einer Doppelhaarnadelnukleinsäure, die aus einem Nukleinsäureeinzelstrang besteht, der Folgendes aufweist:
i. eine erste Haarnadel am 5'-Ende;
ii. eine zweite Haarnadel am 3'-Ende und
iii.eine einzelsträngige Region zwischen dem 5'-Ende und dem 3'-Ende, wobei die einzelsträngige Region an den Vorwärtsprimer oder den Rückwärtsprimer hybridisieren kann;
d. Ligieren des Vorwärts- und Rückwärtsprimers an die entsprechende Doppelhaarnadelnukleinsäure unter Bildung eines inerten zirkulären Produktes, wodurch der Vorwärts- und Rückwärtsprimer aus der Lösung von amplifizierten Zielnukleinsäuren entfernt werden.

12. Verfahren zum Herstellen einer Lösung von amplifizierten Zielnukleinsäuren, die frei von überschüssigen Sonden ist, wobei das Verfahren Folgendes umfasst:
a. Inkontaktbringen eines Zielnukleinsäuren enthaltenden Reaktionsgemisches mit einer ersten und zweiten Sonde, die angrenzend an die Zielnukleinsäure hybridisieren können, und einer wärmebeständigen Ligase in der Gegenwart von Reagenzien, die die Ligation unterstützen;
b. Unterziehen des Reaktionsgemisches einem Thermocycling-Profil, das für Annealing und Ligation der ersten und zweiten Sonde aneinander geeignet ist;
c. nach dem Abschluss des Thermocycling-Profils, Inkontaktbringen des Reaktionsgemisches mit einer Doppelhaarnadelnukleinsäure, die aus einem Nukleinsäureeinzelstrang besteht, der Folgendes aufweist:
i. eine erste Haarnadel am 5'-Ende;
ii. eine zweite Haarnadel am 3'-Ende und
iii. eine einzelsträngige Region zwischen dem 5'-Ende und dem 3'-Ende, wobei die einzelsträngige Region an die erste Sonde oder an die zweite Sonde hybridisieren kann;
d. Ligieren der ersten und zweiten Sonde an die entsprechende Doppelhaarnadelnukleinsäure unter Bildung eines inerten zirkulären Produktes, wodurch die erste und zweite Sonde aus der Lösung von amplifizierten Zielnukleinsäuren entfernt werden.

13. Verfahren zum Bilden einer Bibliothek von Nukleinsäuren, die frei von überschüssigen Adaptor-Molekülen ist, wobei das Verfahren Folgendes umfasst:
a. Inkontaktbringen eines Reaktionsgemisches, das Nukleinsäuren umfasst, mit Adaptor-Molekülen und einer Ligase;
b. Ligieren der Adaptor-Moleküle an die Enden der Nukleinsäuren unter Bildung einer Bibliothek von Nukleinsäuren;
c. Inkontaktbringen des Reaktionsgemisches mit einer Doppelhaarnadelnukleinsäure, die aus einem Nukleinsäureeinzelstrang besteht, der Folgendes aufweist:
i. eine erste Haarnadel am 5'-Ende;
ii. eine zweite Haarnadel am 3'-Ende und
iii. eine einzelsträngige Region zwischen dem 5'-Ende und dem 3'-Ende, wobei die einzelsträngige Region an den Adaptor hybridisieren kann;
d. Ligieren des Adaptors an die Doppelhaarnadelnukleinsäure unter Bildung eines inerten zirkulären Produktes, wodurch der Adaptor aus der Lösung, die die Bibliothek von Nukleinsäuren enthält, entfernt wird.

## Revendications

1. Procédé d'élimination d'oligonucléotides non souhaités à partir d'un mélange réactionnel, le procédé comprenant :
a. la mise en contact du mélange réactionnel avec un acide nucléique en double épingle à cheveux comprenant un brin unique d'acide nucléique ayant :
i. une première épingle à cheveux à l'extrémité 5' ;
ii. une seconde épingle à cheveux à l'extrémité 3' ; et
iii. une région simple brin entre l'extrémité 5' et l'extrémité 3', dans lequel la région simple brin comprend une séquence capable de s'hybrider à l'oligonucléotide à éliminer ;
b. la renaturation de l'oligonucléotide à éliminer à l'acide nucléique en double épingle à cheveux ;
c. la ligation de l'oligonucléotide à éliminer aux extrémités de l'acide nucléique en double épingle à cheveux pour former un produit circulaire inerte, éliminant ainsi l'oligonucléotide non souhaité du mélange réactionnel.

2. Procédé selon la revendication 1, comprenant en outre la mise en contact du mélange réactionnel avec une ligase avant l'étape c.

3. Procédé selon la revendication 1, dans lequel le mélange réactionnel comprend une ligase avant l'étape a.

4. Procédé selon la revendication 1, dans lequel la double épingle à cheveux contient un groupe 5'-phosphate.

5. Procédé selon la revendication 1, dans lequel la région simple brin comprend deux régions de complémentarité à l'oligonucléotide à éliminer encadrant une région centrale unique.

6. Procédé selon la revendication 5, dans lequel la région centrale est un espaceur non nucléotidique.

7. Procédé selon la revendication 5, dans lequel la région centrale comprend des nucléotides inosines.

8. Procédé selon la revendication 5, dans lequel l'oligonucléotide à éliminer comprend une pluralité d'oligonucléotides ayant deux régions constantes encadrant une unique région de code-barres variant parmi la pluralité d'oligonucléotides.

9. Procédé de détection d'une pluralité de cibles dans une pluralité de cellules dans un mélange réactionnel, le procédé comprenant :
a. la liaison aux cibles dans une pluralité de cellules d'une pluralité d'agents de liaison uniques qui sont chacun spécifiques de l'une des cibles ;
b. l'ajout de multiples oligonucléotides de sous-code à chacun des agents liés dans la pluralité de cellules d'une manière ordonnée pendant des cycles successifs de synthèse par division de groupes dans lequel les oligonucléotides de sous-code se renaturent à chaque cycle de manière adjacente à l'oligonucléotide de sous-code d'un cycle précédent par l'intermédiaire d'une région de renaturation, et la liaison covalente des oligonucléotides de sous-code renaturés de manière adjacente les uns aux autres pour créer dans chaque cellule, un code nucléotidique unique ayant pour origine la cellule ;
c. la mise en contact du mélange réactionnel avec un acide nucléique en double épingle à cheveux comprenant un brin unique d'acide nucléique ayant une première épingle à cheveux à l'extrémité 5' ; une seconde épingle à cheveux à l'extrémité 3' ; et une région simple brin entre l'extrémité 5' et l'extrémité 3' capable de s'hybrider aux oligonucléotides de sous-code, et la ligation des oligonucléotides de sous-code en excès à l'acide nucléique en double épingle à cheveux correspondant pour former un produit circulaire inerte, éliminant ainsi les oligonucléotides de sous-code en excès.

10. Procédé selon la revendication 9, dans lequel les oligonucléotides de sous-code comprennent une région de code-barres encadrée par deux régions de renaturation et la région simple brin de l'acide nucléique en double épingle à cheveux comprend un espaceur de longueur égale à la région de code-barres encadrée par deux séquences capables de s'hybrider aux régions de renaturation dans les oligonucléotides de sous-code.

11. Procédé de préparation d'une solution d'acides nucléiques cibles amplifiés exempte d'amorces d'amplification en excès, le procédé comprenant :
a. la mise en contact d'un mélange réactionnel contenant des acides nucléiques cibles avec des amorces d'amplification sens et antisens et une acide nucléique polymérase thermostable en présence de réactifs soutenant la synthèse d'acides nucléiques ;
b. la soumission du mélange réactionnel à un profil de thermocyclage approprié pour la renaturation et l'extension des amorces sens et antisens ;
c. après l'achèvement du profil de thermocyclage, la mise en contact du mélange réactionnel avec une acide nucléique ligase et un acide nucléique en double épingle à cheveux constitué d'un brin unique d'acide nucléique ayant :
i. une première épingle à cheveux à l'extrémité 5' ;
ii. une seconde épingle à cheveux à l'extrémité 3' ; et
iii. une région simple brin entre l'extrémité 5' et l'extrémité 3', dans lequel la région simple brin est capable de s'hybrider à l'amorce sens ou à l'amorce antisens ;
d. la ligation des amorces sens et antisens à l'acide nucléique en double épingle à cheveux correspondant pour former un produit circulaire inerte, éliminant ainsi les amorces sens et antisens de la solution d'acides nucléiques cibles amplifiés.

12. Procédé de préparation d'une solution d'acides nucléiques cibles amplifiés exempte de sondes en excès, le procédé comprenant :
a. la mise en contact d'un mélange réactionnel contenant des acides nucléiques cibles avec une première et une seconde sonde capables de s'hybrider de manière adjacente à l'acide nucléique cible, et une ligase thermostable en présence de réactifs soutenant la ligation ;
b. la soumission du mélange réactionnel à un profil de thermocyclage approprié pour la renaturation et la ligation des première et seconde sondes l'une à l'autre ;
c. après l'achèvement du profil de thermocyclage, la mise en contact du mélange réactionnel avec un acide nucléique en double épingle à cheveux constitué d'un brin unique d'acide nucléique ayant :
i. une première épingle à cheveux à l'extrémité 5' ;
ii. une seconde épingle à cheveux à l'extrémité 3' ; et
iii. une région simple brin entre l'extrémité 5' et l'extrémité 3', dans lequel la région simple brin est capable de s'hybrider à la première sonde ou à la seconde sonde ;
d. la ligation des première et seconde sondes à l'acide nucléique en double épingle à cheveux correspondant pour former un produit circulaire inerte, éliminant ainsi les première et seconde sondes de la solution d'acides nucléiques cibles amplifiés.

13. Procédé de formation d'une banque d'acides nucléiques exempts de molécules adaptatrices en excès, le procédé comprenant :
a. la mise en contact d'un mélange réactionnel comprenant des acides nucléiques avec des molécules adaptatrices et une ligase ;
b. la ligation des molécules adaptatrices aux extrémités des acides nucléiques pour former une banque d'acides nucléiques ;
c. la mise en contact du mélange réactionnel avec un acide nucléique en double épingle à cheveux constitué d'un brin unique d'acide nucléique ayant :
i. une première épingle à cheveux à l'extrémité 5' ;
ii. une seconde épingle à cheveux à l'extrémité 3' ; et
iii. une région simple brin entre l'extrémité 5' et l'extrémité 3', dans lequel la région simple brin est capable de s'hybrider à l'adaptateur ;
d. la ligation de l'adaptateur à l'acide nucléique en double épingle à cheveux pour former un produit circulaire inerte, éliminant ainsi l'adaptateur de la solution contenant la banque d'acides nucléiques.
